Europäisches Patentamt

European Patent Office

Office européen des brevets

(11) Veröffentlichungsnummer: **0 379 710**
**A1**

(12) # EUROPÄISCHE PATENTANMELDUNG

(21) Anmeldenummer: **89123613.5**

(22) Anmeldetag: **21.12.89**

(51) Int. Cl.5: **C12N 15/70, C12N 15/31,**
**A61K 39/02, G01N 33/569**

(30) Priorität: **24.12.88 DE 3843894**

(43) Veröffentlichungstag der Anmeldung:
**01.08.90 Patentblatt 90/31**

(84) Benannte Vertragsstaaten:
**AT BE CH DE ES FR GB GR IT LI LU NL SE**

(71) Anmelder: **BEHRINGWERKE**
**Aktiengesellschaft**
**Postfach 1140**
**D-3550 Marburg 1(DE)**

(72) Erfinder: **Bröker, Michael, Dr.**
**Lindenweg 16**
**D-3550 Marburg(DE)**

(74) Vertreter: **Klein, Otto, Dr. et al**
**Hoechst AG Zentrale Patentabteilung**
**Postfach 80 03 20**
**D-6230 Frankfurt am Main 80(DE)**

(54) **Plasmide mit Translations-Start-Stop-Start Codon-Konfiguration zur Expression von Proteinen in E.coli.**

(57) Die Erfindung betrifft Expressions-Plasmide, die eine wesentliche Expressionssteigerung von Proteinen bewerkstelligen, insbesondere des hitzelabilen E.coli Enterotoxins Untereinheit B (LT-B). Dabei wird der für LT-B kodierenden DNA ein starker Promotor so vorangestellt, daß die sogenannte Shine-Dalgarno-Sequenz GGGA des LT-B Gens dem Translationsstart des LT-B sechs Basenpaare (bp) vorgelagert ist, und wobei das Stopcodon TGA der im natürlichen Fall vorausgehenden LT-A Sequenz erhalten bleibt.

EP 0 379 710 A1

EP 0 379 710 A1

## Plasmide mit Translations-Start-Stop-Start Codon-Konfiguration zur Expression von Proteinen in E.coli

Die Erfindung betrifft Expressions-Plasmide, die eine wesentliche Expressionssteigerung von Proteinen bewerkstelligen, insbesondere des hitzelabilen E.coli Enterotoxins Untereinheit B (LT-B). Dabei wird der für LT-B kodierenden DNA ein starker Promoter so vorangestellt, daß die sogenannte Shine-Dalgarno-Sequenz GGGA des LT-B Gens dem Translationsstart des LT-B sechs Basenpaare (bp) vorgelagert ist, und wobei das Stopcodon TGA der im natürlichen Fall vorausgehenden LT-A Sequenz erhalten bleibt.

Infektionen mit enterotoxinogenen Escherichia coli (E.coli) sind häufig die Ursache einer Diarrhoe bei Menschen und Tieren. Neben dem hitzestabilen E.coli-Enterotoxin verursacht vor allem das hitzelabile E.coli-Enterotoxin (LT) signifikante ökonomische Probleme in der Schweinezucht. Neugeborene Schweine infizieren sich häufig innerhalb weniger Stunden nach der Geburt mit pathogenen E.coli-Stämmen, was zu einer hohen Mortalitätsrate führt. Eine Vakzinierung mit LT-Toxoid könnte dazu beitragen, die Infektionsrate und damit die Ausfälle in Großtierhaltungen zu verringern.

Die Eigenschaften des LT sind dem des Choleratoxins ähnlich. Die A-Untereinheiten beider Toxine beeinflussen die Membranpermeabilität über die Stimulierung der Membran-gebundenen Adenylatcyclase der Epithelzellen des Intestinums. Die Bindung des LT erfolgt über die B-Untereinheit (LT-B) an den $G_M$-Gangliosid-Rezeptor auf der Oberfläche der Mucosazellen des Intestinums. Das LT wird von einer polycistronischen Boten-RNA (mRNA) translatiert, die für die A- und B-Untereinheiten separate Ribosomenbindungsstellen (RBS) = Shine-Dalgarno-Sequenzen (SD) besitzt, was wahrscheinlich Ursache für die unterschiedliche Translationsinitiationsrate von etwa fünf B-Untereinheiten zu einer A-Untereinheit ist. Das Gen für die LT-B wurde von Dallas und Falkow (Nature 288, 499-501, (1980)) sequenziert. Das LT-B-Gen kodiert für ein Protein von 124 Aminosäuren, wobei die aminoterminalen 22 Aminosäuren als Signalsequenz fungieren, die während des Transportes von LT-B durch die Zellmembran abgespalten werden. Das Molekulargewicht des prozessierten LT-B beträgt 11780 Dal. Da LT-B nicht pathogen, aber immunogen ist, wurde im folgenden ein Verfahren entwikkelt, um LT-B in apathogenen E.coli mit Hilfe molekularbiologischer Methoden in großen Mengen produzieren zu können.

Die Synthese des LT-B sollte durch starke, vorteilhafterweise regulierbare Promotoren erfolgen, wie sie der tac-Promoter darstellt (De Boer, H.A. et al. in: Rodriguez et al., Promotors, Structure and Function, Praeger, New York, 1982, 462-481). Andererseits sollte die RBS des LT-B-Gens ausgenutzt werden, um eine hohe Translation der transkribierten mRNA in E.coli zu gewährleisten.

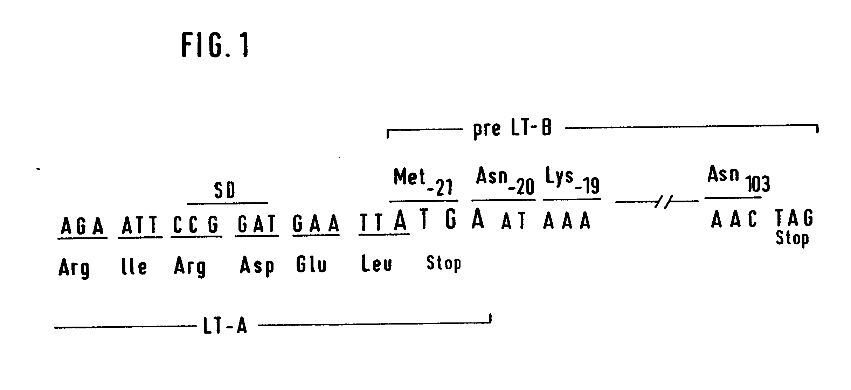

Die Gene der LT-A- und LT-B-Untereinheit überlappen über einen Bereich von vier Nukleotiden (siehe Fig. 1). Das proximale A der Sequenz ATGA ist bei dem LT-A die dritte Base innerhalb des $TTA_{Leu}$-Codons und die letzten drei Basen TGA kodieren für den Translationsstop des LT-A. Hingegen ist das proximale A der Sequenz ATGA die erste Base des $ATG_{Met}$-Codons, das für die Initiation der prä-LT-B-Translation, allerdings im anderen Leseraster, kodiert.

Wir haben gefunden, daß die Expression von Proteinen, insbesondere LT-B wesentlich gesteigert ist, wenn man einen Expressionsvektor einsetzt, der o.g. Situation widerspiegelt. Solche Vektoren besitzen nach einem starken Promotor einen ATG-Start, gefolgt von 5 bis 10 Tripletts, die wiederum eine SD-Sequenz beinhalten; daran schließt sich ein Translations-Stop-Codon TGA an, gefolgt von 1 bis 5 Tripletts bis zum ATG-Start des Polypeptids von Interesse. Möglicherweise tritt die - durch das insertierte TGA-Stop-Triplett ganz unerwartete - Expressionssteigerung durch diese in kurzem Abstand erfolgende "Start-Stop-Restart"-Anordnung ein.

Die Erfindung betrifft folglich Expressionsplasmide, die nach einem starken Promotor ein erstes ATG-Startcodon besitzen, gefolgt von 5 bis 10 für Aminosäuren kodierende Tripletts, wobei eine zweite SD-Sequenz enthalten ist, sich ein Stopcodon anschließt und in kurzem Abstand von 1 bis 5 weiteren Tripletts ein weiteres ATG-Startcodon erreicht wird, auf das die DNA des Polypeptids von Interesse folgt.

Ferner betrifft die Erfindung die Verwendung solcher Plasmide zur Expression von Proteinen, insbesondere LT-B in E.coli und die gentechnischen Verfahren dazu.

Die Erfindung ist schließlich in den Beispielen und den Patentansprüchen beschrieben.

Beispiele

Beispiel 1: Geeignete Ausgangsplasmide

Ausgangspunkt für die Herstellung geeigneter Expressionsvektoren war das Plasmid pOB3 (Bröker, Biotechniques, 6, 734, 1988). Dieses Plasmid ist in E.coli in hoher Kopienzahl replizierbar. Das Ampicillin-Resistenzgen ermöglicht eine Selektion und Fermentation in Gegenwart von ß-Lactam-Antibiotika. 3'-endig vom tac-Promotor befindet sich ein ATG-Translationstriplett, dem einige Restriktionsstellen und weiter 3'-endig die starken ribosomalen Transkriptionsterminatoren $T_1 T_2$ folgen.

Beispiel 2: Konstruktion eines LT-B-Expressionsvektors

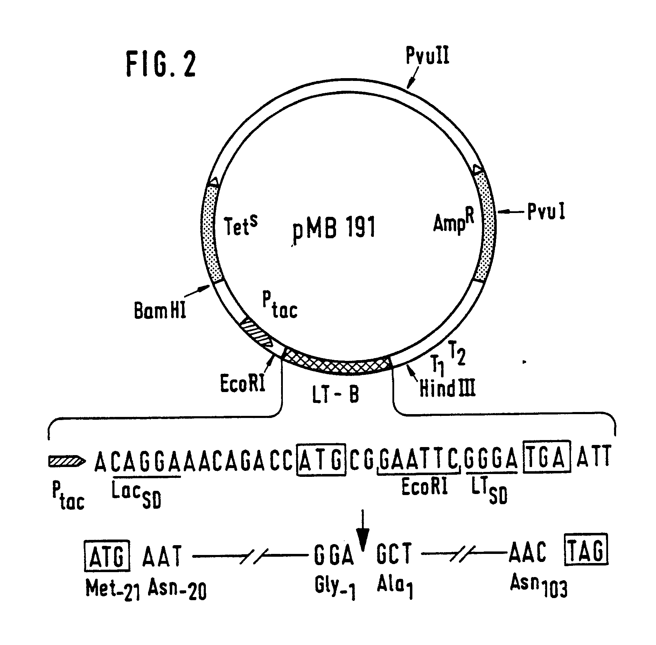

Das Plasmid pOB3 wurde mit EcoRI und HindIII verdaut, und das EcoRI-HindIII DNA-Fragment, das für LT-B codiert, in das Plasmid einligiert. Das neue Plasmid pMB191 (Fig. 2) kodiert somit für die Expression des LT-B, reguliert durch den tac-Promotor. Zusätzlich ist eine Situation geschaffen, in der, bedingt durch das ATG-Codon und die folgenden Tripletts ein Pentapeptid der Struktur Met-Arg-Asn-Ser-Gly in E.coli synthetisiert wird. Vier Basenpaare 3'-endig des TGA-Stop-Codons folgt ein neues ATG-Translationsstartsignal, nunmehr Bestandteil der prä-LT-B-Sequenz.

Durch den kurz auf den Translationsstop wiederholten Translationsstart ist eine Situation geschaffen, die bei der Translation der natürlichen bicistronischen mRNA, die für LT-A und LT-B kodiert, nachempfunden ist. Es erfolgt, kodiert durch pMB191, die Synthese einer mRNA, auf der, ebenso wie bei dem authentischen Gentranskript, die Shine-Dalgarno-Sequenz GGGA dem Translationsstart des LT-B sechs bp vorgelagert ist. Im vorliegenden Beispiel wurde das TGA-Stopcodon noch zusätzlich im LT-B-Lese raster eingebaut, was an sich nicht erforderlich ist.

Beispiel 3: Expression von LT-B

Das neue Plasmid pMB191 wurde in E.coli D29A1 transformiert. Transformierte Zellen wurden auf LB-Medium angezogen und der tac-Promotor mit Isopropylthiogalaktosid (IPTG) induziert. Proteinextrakte von E.coli D29A1 pMB191 wurden in SDS-Polyacrylamidgelen aufgetrennt. Im Vergleich zu Extrakten, die von nicht-induzierten Kulturen stammen, konnte durch eine Coomassie brilliantblau-Färbung eine neue Bande von einem scheinbaren Molekulargewicht von ca. 12000 Dal sichtbar gemacht werden. Durch Inkubation der Zellen in einem Puffer von pH 8,0 mit 10 mM EDTA kann erreicht werden, daß LT-B in den Puffer ausgeschleust wird. Mit Hilfe dieses neuen Expressionssystems kann man große Mengen von immunogenem LT-B in apathogenen E.coli-Stämmen als Ausgangsmaterial für eine Vakzine herstellen. In einem Stamm, der den Genotyp lacI$^Q$ besitzt, kann die Expression reguliert erfolgen. Doch sind auch E.coli-Stämme einsetzbar, in denen LT-B konstitutiv gebildet wird. Die Ausbeute an LT-B kann zusätzlich gesteigert werden, indem statt des üblichen LB-Mediums ein modifiziertes LB-Medium zur Fermentation verwendet wird. Dieses Medium wird wie folgt angesezt: Man gibt 100 ml einer sterilen Lösung von 0,17 M $KH_2PO_4$ und 0,72 M $K_2HPO$ zu einer sterilen Lösung, die 12 g Bacto-Trypton, 24 g Bacto-Hefeextrakt und 4 ml Glycerin mit $H_2O$ auf 900 ml aufgefüllt, enthält.

Im Vergleich zu herkömmlichen Expressionssystemen tritt durch Einsatz von pMB191 eine Steigerung der LT-B Expression um den Faktor 100 ein. Im vorliegenden Fall wird das Protein von Interesse durch Abspaltung der Signalsequenz prozessiert. Allgemein wird man eine solche Sequenz vorvorschalten, um ein "reifes" Protein ohne zusätzlichen Aufwand zu erhalten.

Legende zu Fig. 1 und Fig. 2

Fig. 1:

Ausschnitt aus der DNA-Sequenz des LT-Gens. Übergang vom Protein-kodierenden LT-A zum LT-B Bereich. SD: Shine Dalgarno Sequenz von LT-B.

Fig. 2:

Schematische Darstellung des Vektors pMB191 $P_{tac}$:
tac-Promotor. Amp$^R$: Ampicillinresistenz,
Tet$^s$: Teil des Gens, das Rsistenz gegen Tetracyclin vermittelt.
$T_1 T_2$: Transkriptionsterminator.

$LT_{SD}$: Shine-Dalgarno Sequenz der LT-B Untereinheit. Der Pfeil gibt die proteolytische Spaltung des prä-LT-B an.

**Ansprüche**

1. Expressionsplasmide zur Expression von Proteinen in E.coli mit den aufeinanderfolgenden Elementen
(a) einem starken Promotor,
(b) einem ersten Startcodon,
(c) 3 bis 10 Tripletts, die am 3' Ende die Shine-Dalgarno (SD) Sequenz AGGA oder GGGA oder eine andere als SD funktionelle Sequenz enthalten,
(d) einem Stopcodon,
(e) einem zweiten Startcodon in 1-10 Tripletts Abstand,
die vor der für das Protein von Interesse kodierenden DNA angeordnet sind.

2. Expressionsplasmide nach Anspruch 1, wobei dem Protein von Interesse eine Signalsequenz vorgeschaltet ist.

3. Expressionsplasmide nach Anspruch 1, wobei der tac-Promotor mit anschließender Sequenz

ACAGGAAACAGACC ATG CGG AAT TCG GGA TGA ATT

$Lac_{SD}$          $LT_{SD}$

vor der ATG-Sequenz des zu exprimierenden Proteins eingesetzt wird.

4. Expressionsplasmide nach Anspruch 1, 2 oder 3 wobei die LT-B cDNA Sequenz eingesetzt wird.

5. Verfahren zur gentechnischen Herstellung von Proteinen, dadurch gekennzeichnet, daß ein Expressionsplasmid nach Anspruch 1, 2 oder 3 nach Transformation in E.coli dort zur Expression gebracht wird.

6. Verfahren zur Herstellung von LT-B, dadurch gekennzeichnet, daß ein Expressionsplasmid nach Anspruch 4 nach Transformation in E.coli dort zur Expression gebracht wird.

7. Vakzine, die ein nach Anspruch 5 hergestelltes LT-B enthält.

8. Verwendung von nach Anspruch 5 hergestelltem LT-B zur Vakzinierung von Nutztieren.

9. Diagnostischer Kit, der ein nach Anspruch 5 hergestelltes LT-B enthält.

Patentansprüche für folgende Vertragsstaaten: ES, GR:

1. Verfahren zur Expression von Proteinen in E.coli, dadurch gekennzeichnet, daß ein Expressionsplasmid mit den aufeinanderfolgenden Elementen:
(a) einem starken Promotor,
(b) einem ersten Startcodon,
(c) 3 bis 10 Tripletts, die am 3' Ende die Shine-Dalgarno (SD) Sequenz AGGA oder GGGA oder eine andere als SD funktionelle Sequenz enthalten,
(d) einem Stopcodon,
(e) einem zweiten Startcodon in 1 - 10 Tripletts Abstand,
die vor der für das Protein von Interesse kodierenden DNA angeordnet sind, zur Expression gebracht wird.

2. Verfahren nach Anspruch 1, dadurch gekennzeichnet, daß dem Protein von Interesse eine Signalsequenz vorgeschaltet ist.

3. Verfahren nach Anspruch 1, dadurch gekennzeichnet, daß der tac-Promotor mit anschließender Sequenz

ACAGGAACAGACC ATG CGG AAT TCG GGA TGA ATT

$Lac_{SD}$          $LT_{SD}$

vor der ATG-Sequenz des zu exprimierenden Proteins eingesetzt wird.

4. Verfahren nach Anspruch 1, 2 oder 3, dadurch gekennzeichnet, daß die LT-B cDNA Sequenz eingesetzt wird.

5. Verfahren zur Herstellung eine Vakzine, dadurch gekennzeichnet, daß nach Anspruch 4 hergestelltes LT-B gereinigt und mit üblichen Hilfsstoffen und Adjuvantien versetzt wird.

# FIG.1

pre LT-B ─────────────────────

                                    Met$_{-21}$  Asn$_{-20}$  Lys$_{-19}$            Asn$_{103}$

SD

AGA  ATT  CCG  GAT  GAA  TT A T G  A AT  AAA  ── // ──  AAC  TAG

Arg  Ile  Arg  Asp  Glu  Leu  Stop                                              Stop

───────────── LT-A ─────────────

EP 0 379 710 A1

# FIG. 2

ACAGGAAACAGACC ATG CG GAATTC GGGA TGA ATT

$P_{tac}$   $Lac_{SD}$   EcoRI   $LT_{SD}$

ATG AAT ——//—— GGA GCT ——//—— AAC TAG

Met$_{-21}$ Asn$_{-20}$   Gly$_{-1}$ Ala$_1$   Asn$_{103}$

**EUROPÄISCHER TEILRECHERCHENBERICHT,**
der nach Regel 45 des Europäischen Patentübereinkommens für das weitere Verfahren als
europäischer Recherchenbericht gilt

Europäisches
Patentamt

Nummer der Anmeldung

EP 89 12 3613

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 154 539 (ELI LILLY) <br><br> * Ansprüche 1-4; Seite 5, Zeile 1-Seite 9, Zeile 5; Seite 20, Zeilen 6-31 * <br><br> -- | 1 | C 12 N 15/70 <br> C 12 N 15/31 <br> A 61 K 39/02 <br> G 01 N 33/569 |
| A | EP-A-0 168 322 (PRAXIS BIOLOGIQUES INC.) <br><br> * Ansprüche * <br><br> -- | 4-8 | |
| A | EP-A-0 035 384 (THE REGENTS OF THE UNIVERSITY OF CALIFORNIA) <br><br> * Seite 29, Zeile 7 - Seite 32, Zeile 3 * <br><br> --------- | 1 | |

RECHERCHIERTE
SACHGEBIETE (Int. Cl.4)

C 12 N

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7,9

Unvollständig recherchierte Patentansprüche:

Nicht recherchierte Patentansprüche: 8

Grund für die Beschränkung der Recherche:

Verfahren zur chirurgischen oder therapeutischen Behandlung des menschlichen oder tierischen Körpers (Siehe Art. 52(4) des Europäischen Patentübereinkommens)

| Recherchenort | Abschlußdatum der Recherche | Prüfer |
|---|---|---|
| Den Haag | 30-03-1990 | HUBER-MACK |